# EUROPEAN PATENT APPLICATION

(11) **EP 4 574 035 A1**
(43) Date of publication of application: **25.06.2025**
(21) Application number: 23855205.3
(22) Date of filing: 21.08.2023
(51) Int. Cl.: A61B 5/145, G01D 11/30, A61B 90/00

(54) **INSERTION DEVICE ASSEMBLY FOR INSERTING ANALYTE MONITORING DEVICE**

(30) Priority: 19.08.2022 KR 20220103941; 19.08.2022 KR 20220103942; 19.08.2022 KR 20220103943; 05.04.2023 KR 20230044729; 01.06.2023 KR 20230070998
(71) Applicant: SD Biosensor, Inc., Gyeonggi-do 16690 (KR)
(72) Inventor: LEE, Hyo Keun, Suwon-si, Gyeonggi-do 16690 (KR); PARK, Hyo-Lim, Suwon-si, Gyeonggi-do 16684 (KR); LEE, Won-Chul, Gimhae-si, Gyeongsangnam-do 50990 (KR); LEE, Jun Hwang, Yongin-si, Gyeonggi-do 16999 (KR); JEON, Sangyeun, Yongin-si, Gyeonggi-do 17091 (KR); HONG, Soonmin, Hwaseong-si, Gyeonggi-do 18477 (KR)
(74) Representative: J A Kemp LLP
(86) International application number: PCT/KR2023/012336
(87) International publication number: WO 2024/039239

(57) **Abstract**

According to an embodiment of the present disclosure, there is provided an insertion device assembly comprising: a transmitter; and an applicator including the transmitter mounted therein, wherein the transmitter comprises an upper case; a lower case coupled to the upper case to form an accommodation space therein; a sensor disposed on the lower case; a hollow holder disposed at a center of the lower case and disposed on the sensor; and wherein the applicator comprises an upper housing having an opening formed at a lower portion thereof and having a button on an outer circumference thereof; a cap configured to be mounted on or separated from the upper housing; a lower housing disposed inside the cap and coupled to an opening at an open lower portion of the upper housing to form an inner space with the upper housing; a needle assembly disposed at an upper portion of the transmitter in the inner space and configured to have at least a portion inserted into the holder; a needle carrier disposed in the inner space and configured to linearly move the needle assembly; and a driving unit configured to provide power to the needle carrier.

## Description

### [Technical Field]

The present disclosure relates to an insertion device assembly, and more particularly, to an insertion device assembly for inserting a device for monitoring an analyte in the body.

### [Background Art]

The contents described in this section merely provide background information for the present disclosure and do not constitute conventional technologies.

As the preference for processed foods has recently increased, people are easily exposed to monosaccharides. Due to this environment, diabetes is also increasing rapidly. Diabetic patients may go into shock and, in rare cases, even death when their blood sugar levels drop or rise rapidly. Therefore, diabetic patients need to continuously monitor their blood sugar levels.

In the case of conventional blood sugar measuring devices, an incision is made at the tip of a finger using a lancet, and the diabetic patient's blood that flows through the incision is inserted into a blood sugar analysis device to calculate a blood sugar level.

This type of blood sugar measuring device causes pain and fear for a user. Additionally, it is realistically difficult to cut your finger every hour and make it bleed.

To solve these problems, continuous glucose monitoring (CGM) systems have been developed. When a sensor with a very small thickness and an electronic device (hereinafter, "transmitter") for analyzing an analyte (hereinafter, "blood sugar") measured by the sensor are attached to the skin of a diabetic patient, blood sugar can be continuously measured for a period of one week to ten days.

The sensor of the transmitter is so thin that it is difficult to directly penetrate the skin. Accordingly, there is a need for a configuration (hereinafter, "applicator") of inserting a needle into the skin, inserting a sensor into its gap, and withdrawing the needle.

Meanwhile, according to the literature US 2021-0038131 A1, a user must insert a sensor introducer 106 into the sensor inserter 500. In this case, the sensor introducer must be aligned and inserted into the sensor inserter, and during this process, there is a high risk that the user will feel fatigued. Therefore, it is desirable that an insertion device assembly be configured such that an applicator and a transmitter are integrated with each other from the time of manufacturing.

Further, according to the literature US 2022-0322975 A1, an on-skin assembly 102 is assembled inside an applicator 100 without any sealing structure between an insertion assembly 118 and a sealing element 110, so there is a risk of exposure to contamination in the future.

Further, according to the literature US 2021-0361197 A1, although a needle unit 550 containing a sensor unit 520 is positioned to penetrate the upper and lower portions of a body attachable unit 20, after the body attachable unit 20 is attached to a user's body and the needle unit 550 is separated, the penetrated portions remain open, and thus there is a risk of infection, etc. due to the inflow of water or foreign substances.

In addition, since a sensor probe 521 exposed to the outside of the body attachable unit 20 as one side of the sensor unit 520 and a sensor body unit 522 positioned inside the body attachable unit 20 as the other side of the sensor unit 520 are positioned to be bent at a right angle, there is a risk that the sensor unit 520 composed of a flexible film may be damaged.

In addition, in the case of sterilization of an applicator using sterilization gas, it takes a long time and there is a concem that residues will remain. For this reason, E-beam is sometimes used for sterilization of medical devices, but in this case, there is a concem that it may affect electronic devices.

In addition, in the case of a general applicator, it is difficult to check the inside of the applicator during an assembly process. Thus, there is a concem that the inspection of defective products may not be carried out properly.

In addition, after sterilization of an insertion device assembly, it is important to maintain a sterile state. In this case, there is a problem that a manufacturing cost increases and waste increases due to double or triple packaging to maintain the sterile state.

### [Disclosure]

### [Technical Problem]

Accordingly, the present disclosure aims to provide an insertion device assembly in which an applicator and a transmitter are manufactured integrally and the preparation operation for use is simple.

In addition, the present disclosure aims to provide an insertion device assembly capable of maintaining a sterile state by applying a double sealing structure between an applicator and a transmitter.

In addition, the present disclosure aims to provide an insertion device assembly that is free from malfunction even when sterilized using an E-beam method.

In addition, the present disclosure aims to provide an insertion device assembly capable of identifying defects during a manufacturing process.

In addition, the present disclosure aims to provide an insertion device assembly that is easy to manufacture because a sealing process for maintaining a sterile state is simplified.

In addition, the present disclosure aims to provide an insertion device assembly capable of sealing a portion through which a needle has penetrated after the needle has been separated from a transmitter.

In addition, the present disclosure aims to provide an insertion device assembly that prevents damage to a sensor unit, which is one of the important components inserted into a user's body to analyze the concentration of an analyte in the body.

The problems to be solved by the present invention are not limited to the problems mentioned above, and other problems not mentioned will be clearly understood by those skilled in the art from the description below.

### [Technical Solution]

According to an embodiment of the present disclosure, there is provided an insertion device assembly comprising: a transmitter; and an applicator including the transmitter mounted therein, wherein the transmitter comprises an upper case; a lower case coupled to the upper case to form an accommodation space therein; a sensor disposed on the lower case; a hollow holder disposed at a center of the lower case and disposed on the sensor; and wherein the applicator comprises an upper housing having an opening formed at a lower portion thereof and having a button on an outer circumference thereof; a cap configured to be mounted on or separated from the upper housing; a lower housing disposed inside the cap and coupled to an opening at an open lower portion of the upper housing to form an inner space with the upper housing; a needle assembly disposed at an upper portion of the transmitter in the inner space and configured to have at least a portion inserted into the holder; a needle carrier disposed in the inner space and configured to linearly move the needle assembly; and a driving unit configured to provide power to the needle carrier.

Preferably, the needle assembly comprises a needle configured to penetrate a user's skin; a grip portion coupled to one end of the needle carrier; and a needle injection portion disposed between the needle and the grip portion and configured to be frictionally coupled to the holder.

Further, preferably, the needle injection portion is coupled to the holder when assembling the insertion device assembly, and the needle injection portion is decoupled from the holder when using the insertion device assembly.

Further, preferably, the cap comprises an opening formed on a lower surface thereof; a plurality of ribs, at least some thereof being disposed in the lower housing, extending radially inwardly from an inner circumference of the cap; and a needle accommodation portion supported by the plurality of ribs and configured to surround at least a part of the needle.

Further, preferably, the needle assembly further comprises a cap injection portion having a shape corresponding to a shape of an upper surface of the needle accommodation portion and disposed on the upper surface of the needle accommodation portion, and the cap injection portion is in contact with a lower surface of the lower case.

Further, preferably, the plurality of ribs is two or more and are disposed symmetrically with respect to a center of the needle accommodation portion, the transmitter further comprises an adhesive tape attached to the lower surface of the lower case, the number of which is equal to the number of the plurality of ribs, and the adhesive tape is inserted through the opening.

Further, preferably, the transmitter further comprises a PCB (Printed Circuit Board) accommodated in the accommodation space between the upper case and the lower case and a dummy PCB connecting the sensor and the PCB.

Further, preferably, the transmitter further comprises a packing portion located on the holder and made of an elastic material.

Further, preferably, the transmitter comprises a lower opening located in the lower case and provided with the holder located therein; and an inclined track having an inclined surface formed to be inclined downward from one side of the lower opening, a part of the sensor being placed on the inclined surface.

Further, preferably, insertion portion of the sensor is located in the needle penetrating the holder, and two bent portions forming an obtuse angle are formed between one end of the sensor and the other end thereof by the inclined surface and the needle.

According to an embodiment of the present disclosure, there is provided an insertion device assembly comprising: a transmitter; and an applicator configured to be mounted therein with the transmitter, wherein the insertion device assembly is divided into a first assembly to a fourth assembly according to an assembling order, and the first assembly comprises a cap comprising an open upper portion and an open lower portion; a lower housing disposed in the cap; a lower case of the transmitter, the lower case being disposed on at least a part of the lower housing; a sensor disposed on the lower case' a hollow holder disposed at a center of the lower case and disposed on the sensor; and a needle assembly disposed on the transmitter and configured to allow at least a part thereof to be inserted into the holder.

Preferably, the second assembly further comprises, in the first assembly sterilized with E-beam, a PCB disposed on the lower case; and an upper case coupled to the lower case to form an accommodation space therein.

Further, preferably, the third assembly further comprises, in the second assembly, a needle carrier configured to linearly move the needle assembly; and a driving unit configured to provide power to the needle carrier.

Further, preferably, the fourth assembly further comprises, in the third assembly, an adhesive tape attached to a lower surface of the lower case; and a sealing portion configured to seal the open lower portion of the cap.

Further, preferably, the needle assembly comprises a needle configured to penetrate a user's skin; a grip portion coupled to one end of the needle carrier; and a needle injection portion disposed between the needle and the grip portion and configured to be frictionally coupled to the holder.

Further, preferably, the cap comprises a plurality of ribs, at least some thereof being disposed in the lower housing, extending radially inwardly from an inner circumference of the cap; a needle accommodation portion supported by the plurality of ribs and configured to surround at least a part of the needle; and a cap injection portion having a shape corresponding to a shape of an upper surface of the needle accommodation portion and disposed on the upper surface of the needle accommodation portion, and the cap injection portion is in contact with a lower surface of the lower case.

Further, preferably, the plurality of ribs is two or more and is disposed symmetrically with respect to a center of the needle accommodation portion, the transmitter further comprises an adhesive tape attached to the lower surface of the lower case, the number of which is equal to the number of the plurality of ribs, and the adhesive tape is inserted through the open lower portion of the cap.

According to an embodiment of the present disclosure, there is provided an insertion device assembly comprising: a transmitter; and an applicator including the transmitter mounted therein, wherein the applicator comprises an upper housing having an opening formed at a lower portion thereof and having a button on an outer circumference thereof; a cap configured to be mounted on or separated from the upper housing, with the upper portion and lower portion of the cap being open; a lower housing disposed inside the cap and coupled to an opening at an open lower portion of the upper housing to form an inner space with the upper housing; and a needle assembly comprising a needle configured to penetrate the transmitter, wherein the cap comprises a plurality of ribs, at least some thereof being disposed in the lower housing, extending radially inwardly from an inner circumference of the cap; a needle accommodation portion supported by the plurality of ribs and configured to surround at least a part of the needle; and a cap injection portion having a shape corresponding to a shape of an upper surface of the needle accommodation portion, disposed on the upper surface of the needle accommodation portion and being in contact with a lower surface of the transmitter.

Further, the plurality of ribs is two or more and is disposed symmetrically with respect to a center of the needle accommodation portion, the transmitter further comprises an adhesive tape attached to the lower surface of the transmitter, the number of which is equal to the number of the plurality of ribs, and the adhesive tape is inserted through the open lower portion of the cap.

### [Advantageous Effects]

As described above, according to an embodiment of the present disclosure, the applicator and the transmitter can be manufactured integrally, and the needle protection configuration can be separated at once by simply removing the cap when using the applicator, thereby providing convenience to a user.

In addition, the interiors of the needle and applicator can be maintained in a sterile state by the double sealing structure of the insertion device assembly.

Further, according to an embodiment of the present disclosure, when assembling the insertion device assembly, the PCB is assembled after sterilization by E-beam, and thus there is no concem that the electronic devices mounted on the PCB break down.

In addition, since the cap is made of a transparent material, it is possible to visually confirm whether a seal is formed between the cap injection portion and the transmitter, so there is an advantage that the inspection of defective products is easy.

In addition, since only the sealing portion needs to be attached to the lower portion of the cap after the assembling of the insertion device assembly is completed, the sealing method is simple and thus convenient for manufacturing.

In addition, an elastic packing portion is positioned in the portion where the needle is penetrated and placed in the transmitter, and thus the penetrated portion is sealed after the needle is separated, thereby preventing the inflow of water or foreign substances and the infection caused by them.

In addition, the sensor is mounted using the track structure inside the case forming the transmitter, and thus the bending portion of the sensor is formed smoothly, thereby preventing damage to the sensor.

### [Brief Description of the Drawings]

FIG. 1 is a front perspective view of an insertion device assembly according to an embodiment of the present disclosure.
FIG. 2 is an exploded perspective view of an insertion device assembly according to an embodiment of the present disclosure.
FIG. 3 is a full and exploded perspective view of a transmitter according to an embodiment of the present disclosure.
FIG. 4 illustrates an interior of a cap according to an embodiment of the present disclosure.
FIG. 5 is a front perspective view of a lower housing according to an embodiment of the present disclosure.
FIG. 6 is a flowchart of a method of assembling an insertion device assembly according to an embodiment of the present disclosure.
FIG. 7 illustrates an assembly sequence of an insertion device assembly according to an embodiment of the present disclosure.
FIGS. 8 to 11 explain the states in an assembling process of an insertion device assembly according to an embodiment of the present disclosure.
FIG. 12 is an entire perspective view of a connection structure of a PCB, a sensor, and a dummy PCB located inside a transmitter according to an embodiment of the present disclosure, viewed from below.
FIG. 13 is an exploded perspective view of a connection structure of a PCB, a sensor, and a dummy PCB located inside a transmitter according to an embodiment of the present disclosure, viewed from below.
FIG. 14 and FIG. 15 are perspective views each illustrating a track structure of a lower case and a sensor installed therein according to an embodiment of the present disclosure.
FIGS. 16 and 17 are longitudinal cross-sectional views illustrating the shape of a sensor when a needle assembly is not coupled to a transmitter and the shape of a sensor when a needle assembly is coupled to a transmitter, respectively, according to an embodiment of the present disclosure.
FIG. 18 explains the coupling of a needle assembly and a transmitter according to another embodiment of the present disclosure.

### [Detailed Description of Preferred Embodiments]

Hereinafter, some embodiments of the present disclosure will be described in detail with illustrative drawings. When adding reference numerals to components of each drawing, it should be noted that identical components are given the same numerals as much as possible even if they are shown on different drawings. In addition, in the description of the present disclosure, when it is determined that a specific description of a related known configuration or function may obscure the gist of the present disclosure, a detailed description thereof will be omitted.

In the description of the components of an embodiment according to the present disclosure, symbols such as first, second, i), ii), a), b), etc. may be used. These symbols are only intended to distinguish one component from other components, and the nature, order, or sequence of the components are not limited by these symbols. When a part of the specification is said to "include" or "comprise" a component, this means that it may include other components rather than excluding them unless explicitly stated otherwise.

In the present disclosure, the "insertion device assembly" refers to an assembly in which an applicator and a transmitter are assembled.

Additionally, in the present disclosure, the "distal position" refers to a position that is relatively farther from the skin than the "proximal position." Meanwhile, the proximal position and distal position are concepts of relative positions, and it may be understood that an element is proximal to one element while also being distal to another element.

Additionally, in the present disclosure, the "fire" refers to an action by a user to activate an applicator, causing a needle to be inserted into the user's skin.

Additionally, in the present disclosure, the "retraction" refers to a process in which the needle returns to its original position after being inserted into the user's skin.

Additionally, in the present disclosure, the "upward" refers to a direction from the proximal position to the distal position.

Additionally, in the present disclosure, the "downward" refers to a direction from the distal position to the proximal position.

Additionally, in the present disclosure, the "horizontal" refers to a plane that contacts an applicator when the applicator is used, and refers to a skin surface. In this disclosure, the curvature of the skin surface is not considered, and an explanation is given assuming that the user's skin surface is flat.

### 1. Description of applicator

### 1.1 Structure of applicator

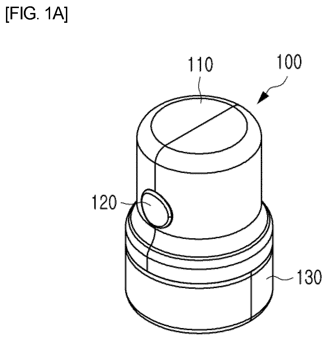

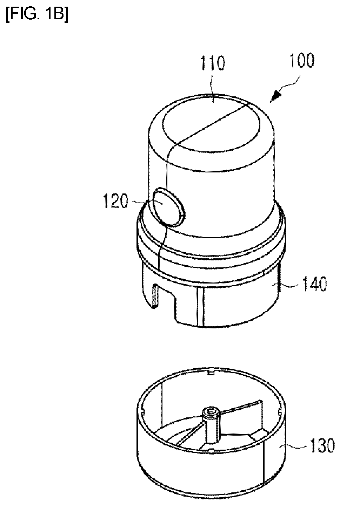

FIG. 1 is a front perspective view of an insertion device assembly according to an embodiment of the present disclosure.

Specifically, FIG. 1A shows a state in which a cap 130 is mounted, and FIG. 1B shows a state in which the cap 130 is separated.

An applicator 100 according to an embodiment of the present disclosure includes an upper housing 110, a button 120 configured to be inserted and pressed into an outer circumference of the upper housing 110, and a cap 130 detachable from the upper housing 110 (refer to FIG. 1A).

When the cap 130 of the applicator 100 is separated from the upper housing 110, a part of a lower housing 140 is revealed (refer to FIG. 1B). When the cap 130 is separated, the insertion device assembly is ready for use by a user.

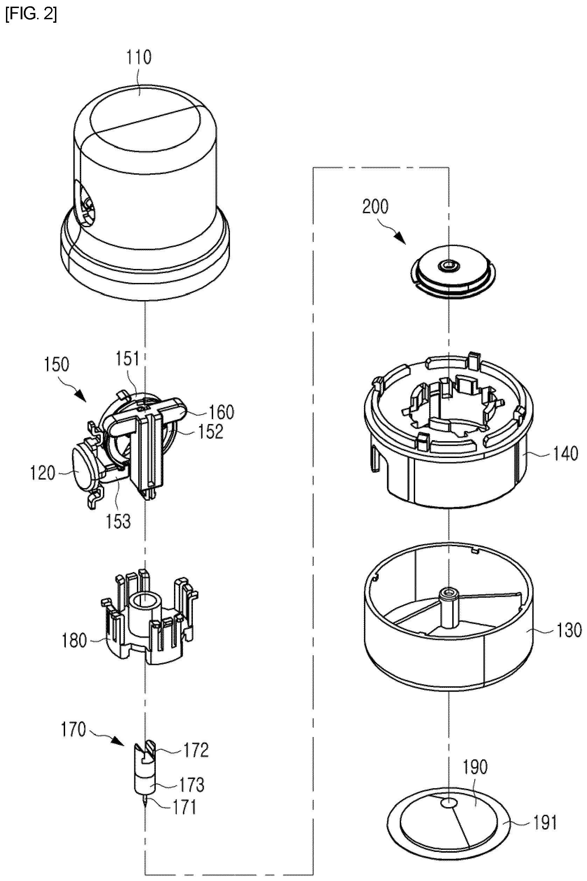

FIG. 2 is an exploded perspective view of an insertion device assembly according to an embodiment of the present disclosure.

Referring to FIG. 2, the applicator 100 according to an embodiment of the present disclosure may include, all or some of a driving unit 150, a needle carrier 160, a needle assembly 170, a transmitter holder 180, a desiccant 190, and a sealing unit 191 in addition to the upper housing 110, the button 120, the cap 130, and the lower housing 140.

The upper housing 110 is configured to be gripped by the user, and, preferably, has an approximately cylindrical shape. Meanwhile, the lower portion of the upper housing (110) is open.

The button 120 is placed and provided on the outer circumference of the upper housing 110, and is configured to be pressed toward the inside of the upper housing 110.

The cap 130 is configured to be mounted on or separated from the upper housing 110, and can protect the interior of the applicator 100 from extemal contaminants or impacts. It is preferable that the cap 130 has an approximately cylindrical shape.

The lower housing 140 is disposed inside the cap 130. The lower housing 140 is configured to form a space inside by coupling with the lower portion of the upper housing 110. When using the applicator 100, the lower housing 140 is configured to come into direct contact with the user's skin.

The driving unit 150 is disposed in an intemal space formed by the upper housing 110 and the lower housing 140. The driving unit 150 is configured to move a transmitter 200 and the needle assembly 170 linearly along the length direction of the applicator 100. For this purpose, the driving unit 150 includes all or some of a spring 151, a wheel 152, and a locking member 153.

The spring 151 is a power source that provides power to the needle carrier 160 so that the needle carrier 160 can move in a straight line. For this purpose, it is preferable that the spring 151 is a wound reel spring. The spring 151 is assembled in a compressed state in the initial state. Meanwhile, when a fastener that was fixing the compression of the spring 151 by the pressurization of the button 120 is released, the spring 151 can be tensioned to return to its original state. Thus, one end of the spring 151 rotates. That is, it may be a kind of Scotch Yoke or double slider crank mechanism principle in which the rotational motion of the spring 151 is converted into the linear motion of the needle carrier 160 and the needle assembly 170.

One side of the wheel 152 is connected to one end of the spring 151, and is thus configured to rotate together with the rotation of the spring 151. A groove for coupling with the locking member 153 may be formed on the outer circumference of the wheel 152.

The locking member 153 is configured such that one end thereof is coupled to the button 120 and the other end thereof is connected to the wheel 152. In this case, a groove is formed at the other end of the locking member 153, so that it may engage with the groove of the wheel 152. As a result, the rotation of the wheel 152 is prevented, and further, the tension of the spring 151 is prevented.

When the button 120 moves toward the inside of the applicator 100, the locking member 153 also moves in conjunction with the button 120. Thus, the engagement between the other end of the locking member 153 and the groove of the cap 130 is released, and the wheel 152 may rotate together due to the tension and rotation of the spring 151.

The needle carrier 160 is configured to move linearly within the upper housing 110 and the lower housing 140. In this case, the linear movement of the needle carrier 160 may be induced by the rotational movement of the driving unit 150. For this purpose, it is preferable that the needle carrier 160 has an overall T-shape. The needle carrier 160 may be coupled to the other side of the wheel 152 to be subject to the rotational movement of the driving unit 150.

The needle assembly 170 is placed in an intemal space formed by the upper housing 110 and the lower housing 140. The needle assembly 170 is coupled to the lower end of the needle carrier 160, and is thus configured to move linearly together with the linear movement of the needle carrier 160.

The needle assembly 170 includes all or some of a needle 171, a grip portion 172, and a needle injection portion 173.

The needle 171 is configured to penetrate the user's skin.

The grip portion 172 is configured to be coupled to the lower portion of the needle carrier 160. The needle 171 and the grip portion 172 may be configured as one integral body, and preferably made entirely of a metal material, but the disclosure is not necessarily limited thereto.

The needle injection portion 173 is disposed between the needle 171 and the grip portion 172. The needle injection portion 173 is made of a different material from the needle 171 and the grip portion 172, and, preferably, may be made of rubber. For this purpose, it is preferable that the needle injection portion 173 be double-injected when manufacturing the needle assembly 170.

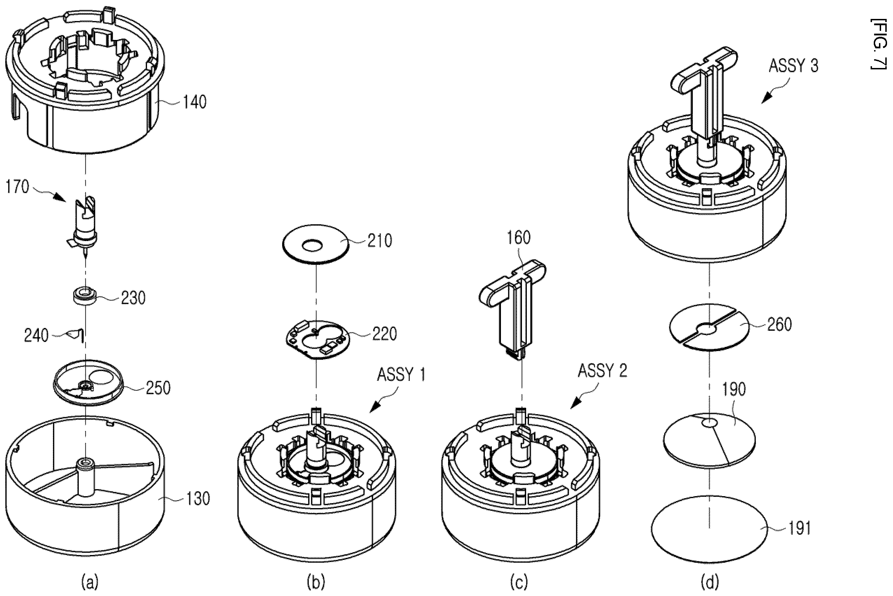

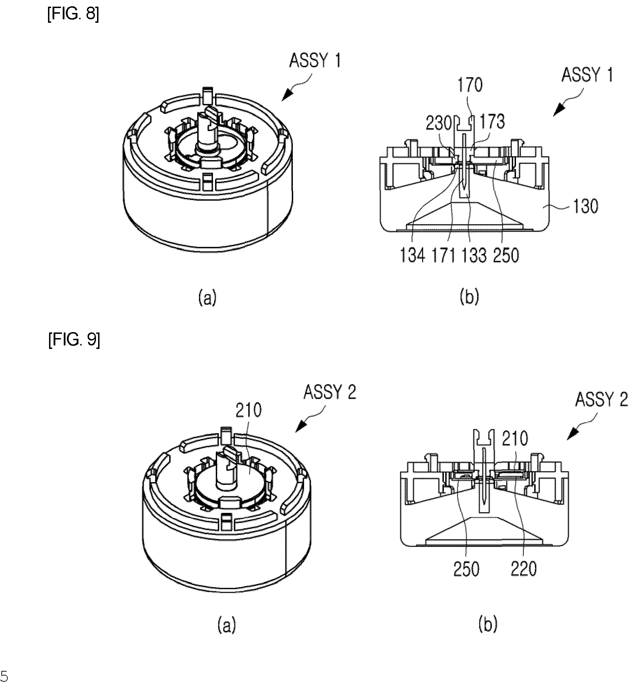

Meanwhile, when manufacturing the insertion device assembly, the needle injection portion 173 is inserted into the interior of the transmitter 200. In this case, since the needle injection portion 173 is made of rubber, a sealing structure may be formed with the transmitter 200. In relation to this, it will be described in detail with reference to FIGS. 7 and 8.

According to an embodiment of the present disclosure, the needle assembly 170 may be prevented from rotating when coupled with the transmitter 200 through the needle injection portion 173.

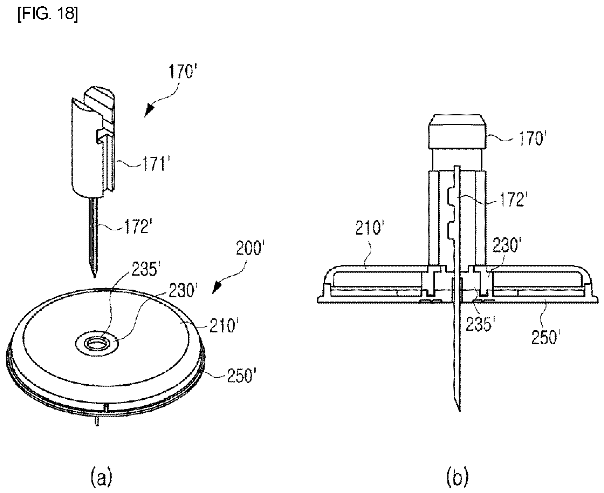

Meanwhile, according to another embodiment of the present disclosure, instead of providing a needle assembly 170' with a separate needle injection portion, at least a part of the upper surface of a transmitter 200' is formed as an elastic part, so that when a needle 171' is coupled with an upper case 210' of the transmitter 200', a sealing structure is formed while preventing rotation of the needle 171' (refer to FIG. 18). In relation to this, it will be described in detail with reference to FIG. 18.

The transmitter holder 180 is disposed in an intemal space formed by the upper housing 110 and the lower housing 140. The transmitter holder 180 is coupled to the transmitter, and is thus configured to move the transmitter 200 linearly from a distal position to a proximal position. The transmitter holder 180 is pressed from the distal to the proximal position by the needle carrier 160. Meanwhile, the transmitter holder 180 does not move from the proximal position to the distal position, but may only move from the distal position to the proximal position.

The desiccant 190 is inserted into the interior of the cap 130 through the bottom surface of the cap 130, and is configured to dehumidify the interior of the applicator 100.

The sealing portion 191 is attached to the open bottom surface of the cap 130, and is configured to block the inside and outside of the applicator 100.

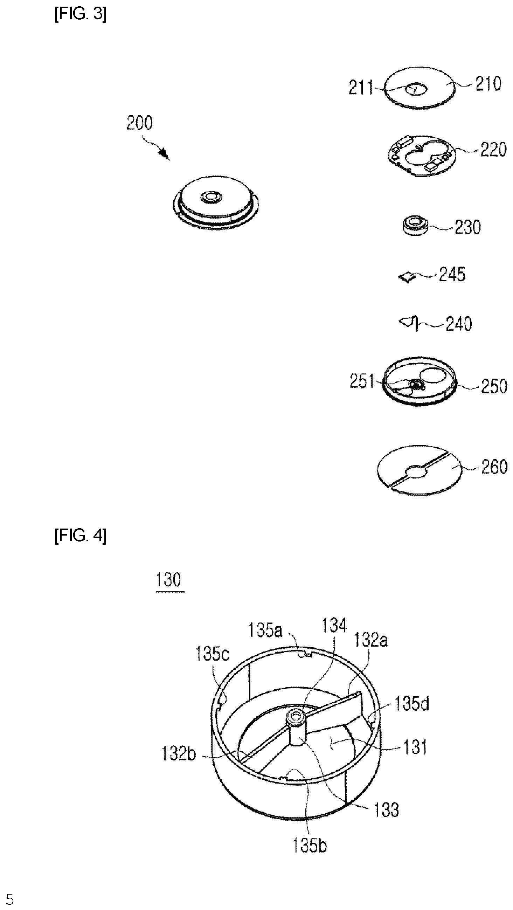

FIG. 3 is a full perspective view and an exploded perspective view of a transmitter according to an embodiment of the present disclosure.

A transmitter 200 according to an embodiment of the present disclosure is configured to be attached to a user's skin, analyze the concentration of an analyte in the user's body, and transmit information about the concentration to an extemal device. The transmitter 200 may be manufactured by being inserted into the interior of the applicator 100 during the production process of the insertion device assembly.

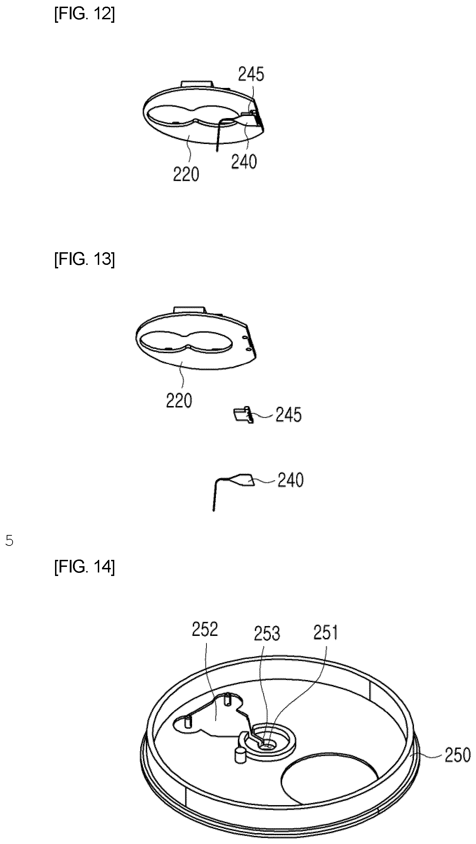

Referring to FIG. 3, the transmitter 200 includes all or some of an upper case 210, a PCB 220, a holder 230, a sensor 240, a dummy PCB 245, a lower case 250, and an adhesive tape 260. Detailed configurations will be described with reference to FIGS. 12 to 17.

FIG. 4 illustrates an interior of a cap according to an embodiment of the present disclosure.

Referring to FIG. 4, the cap 130 according to an embodiment of the present disclosure may be composed of one or more materials among ABS (Acrylonitrile butadiene styrene co-polymer) resin, PC (Polycarbonate) resin, and PE (Polyethylene) resin, but any material commonly used in medical devices such as applicators may be applied without limitation. For example, PC resin and ABS resin may be used in combination, but this may be appropriately changed depending on the designer's choice.

The cap 130 includes all or some of an opening 131, ribs 132a and 132b, a needle accommodation portion 133, a cap injection portion 134, and a coupling projections 135a, 135b, 135c, and 135d.

The opening 131 is formed on the bottom surface of the cap 130.

The ribs 132a and 132b are formed to extend radially inward from the inner circumference of the cap 130. According to an embodiment of the present disclosure, the two ribs 132a and 132b are formed symmetrically, but the disclosure is not necessarily limited thereto, and the number and shape thereof may be appropriately changed according to the designer's choice.

Since the ribs 132a and 132b are arranged along the diameter of the cap 130, the cap 130 may have a bisected shape when viewed from the bottom or top thereof. That is, the cap 130 may have an approximately θ shape.

The needle accommodation portion 133 is formed at the center of the diameter of the cap 130, and is formed to accommodate the needle 171. For this purpose, the needle accommodation portion 133 may have a cylindrical shape that includes an accommodation space therein and has an open upper surface.

The needle accommodation portion 133 is supported by one rib 132a and another rib 132b.

Since the needle accommodation portion 133 is formed integrally with the cap 130, a needle protection configuration may be separated at once by simply separating the cap 130 when using the applicator 100, which provides an advantage of convenience for the user.

The cap injection portion 134 is formed on the upper surface of the needle accommodation portion 133 and comes into contact with the lower surface of the lower case 250 of the transmitter 200. Meanwhile, the cap injection portion 134 may be formed by double injection on the needle accommodation portion 133 during the manufacturing process of the cap 130, but the disclosure is not necessarily limited thereto.

Meanwhile, when manufacturing the insertion device assembly, the cap injection portion 134 comes into contact with the bottom surface of the transmitter 200. In this case, the cap injection portion 134 is made of rubber, so that a sealing structure may be formed with the transmitter 200. In relation to this, it will be described in detail with reference to FIGS. 7 and 8.

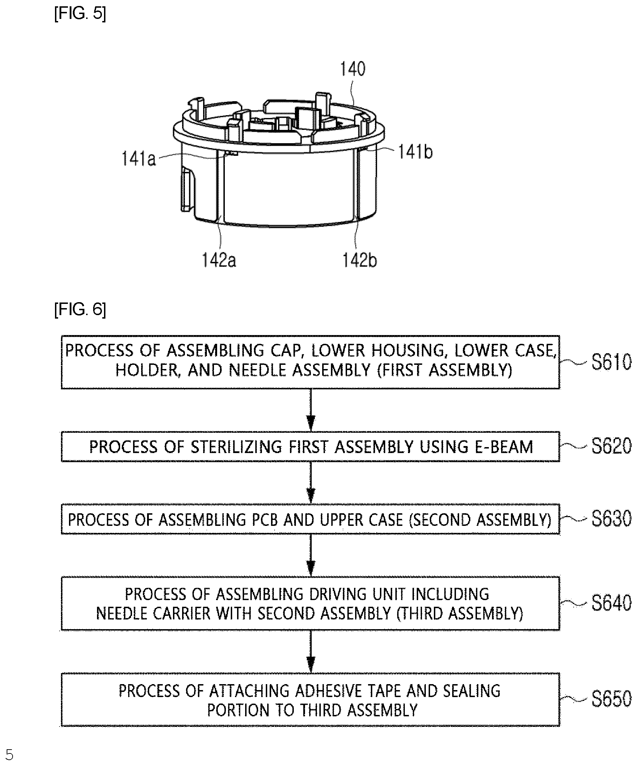

One or more coupling projections 135a, 135b, 135c, and 135d are formed to protrude radially inward from the inner circumference of the cap 130. One or more coupling projections 135a, 135b, 135c, and 135d are configured to be coupled with the outer circumference of the lower housing 140. In relation to this, it will be described in detail with reference to FIG. 5.

FIG. 5 is a front perspective view of a lower housing according to one embodiment of the present disclosure.

Referring to FIG. 5, the lower housing 140 according to an embodiment of the present disclosure includes one or more coupling grooves 141a and 141b formed on the outer circumference thereof.

The coupling grooves 141a and 141b are formed to be recessed radially inward from the outer circumference of the lower housing 140, and may be placed and coupled with the coupling projections 135a, 135b, 135c, and 135d. In this case, the number of the coupling grooves 141a and 141b is formed to be equal to the number of the coupling projections 135a, 135b, 135c, and 135d. Meanwhile, although it is shown in the present disclosure that two coupling grooves 141a and 141b are formed, it should be noted that two more coupling grooves are formed on unseen sides.

Guide grooves 142a and 142b may be further formed on one side of the coupling grooves 141a and 141b. The guide grooves 142a and 142b are formed to be recessed radially inward from the outer circumference of the lower housing 140, but extends along the height direction of the lower housing 140. Meanwhile, it is preferable that the widths of the coupling grooves 141a and 141b are formed to be larger than the widths of the coupling projections 135a, 135b, 135c, and 135d. Thus, the coupling projections 135a, 135b, 135c, and 135d may move up and down within the coupling grooves 141a and 141b.

As an example, the guide grooves 142a and 142b may be formed continuously on the left side of the coupling grooves 141a and 141b. In this case, the coupling projections 135a, 135b, 135c, and 135d are coupled to the coupling grooves 141a and 141b, move to the left side along the widths of the coupling grooves 141a and 141b and the guide grooves 142a and 142b, and then move downward along the height direction of the guide grooves 142a and 142b, so that the coupling projections 135a, 135b, 135c, and 135d may be separated toward the outside of the lower housing 140. That is, the user may release the coupling of the cap 130 and the lower housing 140 by rotating the cap 130 toward the right side along the outer circumference of the lower housing 140 and then moving the cap 130 downward along the height direction of the lower housing 140.

Meanwhile, according to an embodiment of the present disclosure, it is described that the lower housing 140 and the cap 130 may be coupled or decoupled through the coupling projections 135a, 135b, 135c, and 135d and the coupling grooves 141a and 141b, but the disclosure is not necessarily limited thereto. For example, the lower housing (140) and the cap (130) may be coupled by hook coupling, screw coupling, etc. Alternatively, the two may be coupled by the combination of the opening and closing methods of a wing member (not shown) provided on one of the cap 130 and the lower housing 140 and a hook (not shown) provided on the other one thereof.

### 1.2. Method of assembling applicator

FIG. 6 is a flowchart of a method of assembling an insertion device assembly according to an embodiment of the present disclosure. FIG. 7 illustrates an assembly sequence of an insertion device assembly according to an embodiment of the present disclosure. FIGS. 8 to 11 explain the states in an assembling process of an insertion device assembly according to an embodiment of the present disclosure.

A method of assembling and sterilizing an insertion device assembly according to an embodiment of the present disclosure will be described with reference to FIGS. 6 to 11.

First, the cap 130, the lower case 250, the sensor 240, the holder 230, the needle assembly 170, and the lower housing 140 are assembled in that order (S610, (a) of FIG. 7). Hereinafter, the assembly of the cap 130, the lower case 250, the sensor 240, the holder 230, the needle assembly 170, and the lower housing 140 is referred to as the first assembly ASSY 1.

The first assembly ASSY 1 is illustrated in FIG. 8. (a) of FIG. 8 is a front perspective view of the first assembly ASSY 1, and (b) of FIG. 8 is a cross-sectional view of the first assembly ASSY 1. Referring to (b) of FIG. 8, the needle injection portion 173 is pressed into the holder 230, and the two are frictionally coupled. The needle injection portion 173 can prevent contaminants from flowing into the needle 171 through the holder 230. Meanwhile, when the applicator 100 is fired by the user and then the needle assembly 170 is retracted, the transmitter 200 stays at a distal position. Accordingly, the needle injection portion 173 inserted into the holder 230 is separated from the holder 230.

In addition, the cap injection portion 134 comes into contact with the bottom surface of the lower case 250 to form a sealing structure. In this case, due to the coupling of the cap 130 and the lower housing 140, the cap injection portion 134 may be supported while being in contact with the bottom surface of the lower case 250.

It is preferable that at least a part of the cap 130 be made of a transparent material. Thus, it is possible to visually confirm whether a sealing structure is formed between the cap injection portion 134 and the transmitter 200, thereby having an advantage in making it easy to inspect whether there are any defective products that are not sealed.

According to the above description, the insertion device assembly according to an embodiment of the present disclosure has an advantage in that the interior of the insertion device assembly can be protected from contaminants by forming a double sealing structure during assembly. In the case of the needle 171, since it is a part that directly penetrates the user's skin, it is very important to maintain a sterile state, and the double sealing structure of the insertion device assembly according to the present disclosure is significantly effective in maintaining this sterile state.

Thereafter, the first assembly ASSY 1 is sterilized by irradiating the first assembly ASSY 1 with E-beam (S620). Since the PCB 220 is mounted thereon with electronic devices, there is a risk that some functions may be lost when it is directly exposed to the E-beam. Meanwhile, according to the present disclosure, since sterilization by E-beam is performed in a state of the first assembly ASSY 1 in which the PCB 220 is not assembled, there is no concem that the electronic devices mounted on the PCB 220 will break down.

In the first assembly ASSY 1, the PCB 220, the upper case 210, and the adhesive tape 260 are assembled (S630, (b) of FIG. 7). Hereinafter, the assembly formed according to step S630 is referred to as the second assembly ASSY 2.

The second assembly ASSY 2 is illustrated in FIG. 9. (a) of FIG. 9 is a front perspective view of the second assembly ASSY 2, and (b) of FIG. 9 is a cross-sectional view of the second assembly ASSY 2.

Referring to (a) and (b) of FIG. 9, the upper case 210 is coupled to the lower case 250. In this case, the upper case (210) and the lower case 250 may be coupled by either ultrasonic fusion or UV bonding.

Thereafter, in the second assembly ASSY, the needle carrier 160 and the driving unit 150 are assembled (S640, (c) of FIG. 7). Meanwhile, it is illustrated in FIGS. 7, 10 and 11 that only the needle carrier 160 is coupled, but this is for convenience of explanation, and it should be understood that the driving unit 150 coupled to the needle carrier 160 is also assembled together. Hereinafter, the assembly formed according to step S640 is referred to as a third assembly ASSY 3.

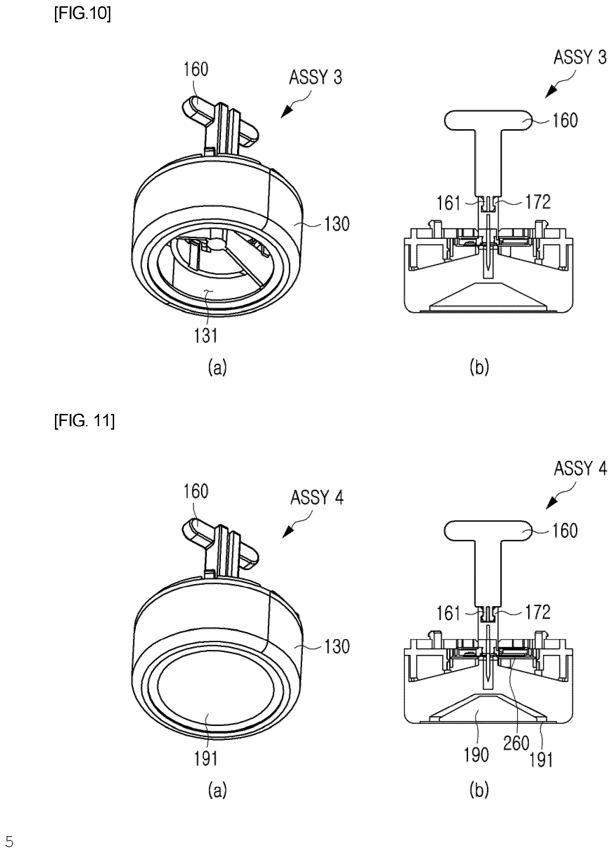

The third assembly ASSY 3 is illustrated in FIG. 10. (a) of FIG. 10 is a bottom perspective view of the third assembly ASSY 3, and (b) of FIG. 10 is a cross-sectional view of the third assembly ASSY 3.

Referring to (a) of FIG. 10, the opening 131 of the cap 130 is still open.

Further, referring to (b) of FIG. 10, the needle carrier 160 and the needle assembly 170 may be coupled by coupling a coupling arm 161 formed at the bottom of the needle carrier 160 and a grip portion 172 of the needle assembly 170. In this case, a snap-fit method may be adopted for the coupling of the connecting arm 161 and the grip portion 172, but the disclosure is not necessarily limited thereto, and a screw coupling, etc. may be adopted instead.

Thereafter, an adhesive tape 260, a desiccant 190, and a sealing portion 191 are sequentially coupled to the third assembly ASSY 3) (S650, (d) of FIG. 7). Hereinafter, the assembly formed according to step S650 is referred to as the fourth assembly ASSY 4.

The fourth assembly ASSY 4 is illustrated in FIG. 11. (a) of FIG. 11 is a bottom perspective view of the fourth assembly ASSY 4, and (b) of FIG. 11 is a cross-sectional view of the fourth assembly ASSY 4.

Referring to (b) of FIG. 11, the adhesive tape 260 is attached to the bottom surface of the lower case 250. In this case, as described above, the adhesive tape 260 may be inserted into the right and left openings 131 of the cap 130 in a bisected state, respectively.

When the adhesive tape 260 is attached, the desiccant 190 is inserted into the cap 130, and the cap 130 is sealed by the sealing portion 191.

Meanwhile, according to an embodiment of the present disclosure, the insertion device assembly has an advantage of being easy to seal because only the sealing portion 191 needs to be attached to the lower portion of the cap 130 during assembly.

Meanwhile, in the present disclosure, after the fourth assembly ASSY 4 is assembled, the upper housing 110 provided with the button 120 is further assembled, but a related description or detailed drawing will be omitted in the present disclosure.

### 1.3. Sterilization test results of applicator and transmitter

As a result of sterilizing an insertion device assembly according to one embodiment or another embodiment of the present disclosure, it was confirmed that proliferation of microorganisms was not observed.

Specifically, the test method was performed according to ISO11737-2:2019, Sterilization of health care products - Microbiological methods-Part 2: Tests of sterility performed in the definition, validation, and maintenance of the sterilization process.

Further, instruments and equipment such as an incubator, a low-temperature incubator, a high-pressure steam sterilizer, and a clean bench were used.

Further, in a soybean casein digest medium (TSB), microorganisms were cultured at 30 to 35°C for 14 days and at 20 to 25°C for 14 days, for a total of 28 days.

In this case, when the irradiation dose of E-beam was 25 kGy and 35 kGy, respectively, the proliferation of microorganisms was confirmed after the culture of microorganisms.

As a result of using the above-mentioned test method, it was confirmed that no microorganisms proliferated in all cases where the irradiation dose of E-beam was 25 kGy and 35 kGy. That is, when the assembling and sterilizing method of the insertion device assembly according to embodiments of the present disclosure is adopted, it can be seen that sterilization effects and contamination prevention effects from extemal contaminants are very excellent.

### 2. Description of Transmitter

Hereinafter, with reference to the attached FIG. 3 and FIGS. 12 to 17, a configuration of a transmitter 200 of an insertion device assembly according to an embodiment of the present disclosure will be described in detail.

As described above, FIG. 3 is a full perspective view and an exploded perspective view of a transmitter 200 according to an embodiment of the present disclosure.

Referring to FIG. 3, the transmitter 200 includes all or some of an upper case 210, a PCB 220, a holder 230, a sensor 240, a dummy PCB 245, a lower case 250, and an adhesive tape 260.

The upper case 210 and the lower case 250 are coupled vertically to form an appearance of the transmitter 200, and a predetermined accommodation space is formed inside the transmitter 200.

An upper through hole 211 is formed at the center of the upper case 210, and a lower through hole 251 is formed at the center of the lower case 250.

A cylindrical holder 230 is located between the upper through hole 211 and the lower through hole 251, and a needle 171 may be disposed to penetrate the upper case 210 and the lower case 250 while a needle injection portion 173 is pressed into the holder 230.

The PCB 220 is a printed circuit board, and is accommodated in the intemal accommodation space between the upper case 210 and the lower case 250. Each electronic component is mounted on the PCB 220 to analyze the concentration of the user's body analyte detected by the sensor 240 and transmit information about the concentration to an extemal device.

The sensor 240 is located under the holder 230 in the intemal accommodation space of the transmitter 200.

One side of the sensor 240 is extended to the outside of the transmitter 200 through the lower through hole 251 of the transmitter 200, placed within the needle 171 and inserted into the user's body by firing, and the other side thereof is connected to the lower portion of the PCB 220 inside the transmitter 200.

Hereinafter, one portion of the transmitter 200 that is inserted into the user's body from the outside may be referred to as an insertion portion, and the other portion of the transmitter 200 that is connected to the PCB 220 from the inside may be referred to as a connection portion.

The dummy PCB 245 is described with reference to FIGS. 12 and 13. FIGS. 12 and 13 are a full perspective view and an exploded perspective view of a connection structure of the PCB 220, the sensor 240, and dummy PCB 245 as viewed from below.

The dummy PCB 245 is located between the sensor 240 and the PCB 220. The dummy PCB 245 is also composed of a small printed circuit board, and electrically connects the sensor 240 with electronic components mounted on the PCB 220.

Unlike the PCB 220, the dummy PCB 245 does not have any component mounted thereon, and only includes a printed circuit for electrically connecting the sensor 240 and the PCB 220.

Specifically, referring to FIG. 12 and FIG. 13, the dummy PCB 245 is located under the PCB 220 and on the sensor 240. One side of the dummy PCB 245 is in contact with the connection portion of the sensor 240, and the other side thereof is in contact with the contact point on the peripheral side of the PCB 220, thereby physically and electrically connecting the sensor 240 and the PCB 220.

The sensor 240 and the dummy PCB 245 may be connected to each other by ultrasonic fusion or anisotropic conductive film (ACF) bonding, and the dummy PCB 245 and the PCB 220 may be connected to each other by soldering.

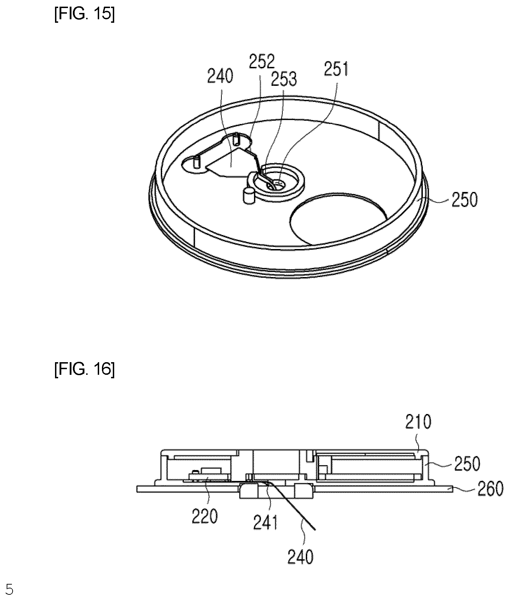

FIGS. 14 and 15 are perspective views of the lower case 250, illustrating a track structure located on the inner surface of the lower case 250 and an appearance of the sensor (240) being installed therein.

Referring to FIG. 14, a fixed track 252 is located on the upper surface of the lower case 250.

The fixed track 252 is formed in a concave and stepped shape with one side of the lower case 250 corresponding to the connection portion side of the sensor 240, and the sensor 240 may be fixedly installed in the lower case 250 by fitting the connection portion of the sensor 240 into the fixed track 252.

An inclined track 253 is located at one side of the fixed track 252. The inclined track 253 is formed in a shape in which the side of the lower through hole 251 of the fixed track 252 is inclined downward.

Referring to FIG. 15, when the sensor 240 is fitted into the fixed track 252, a portion between the connection portion and insertion portion of the sensor 240 is placed on the inclined track 253.

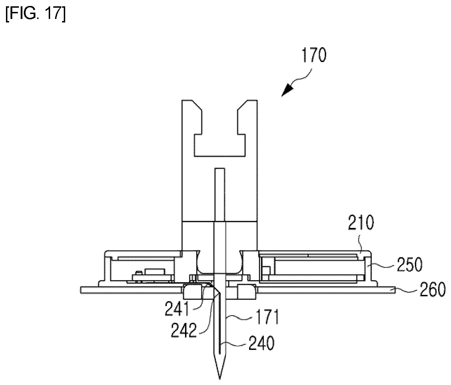

FIG. 16 is a longitudinal cross-sectional view illustrating the state of the sensor 240 when the needle assembly 170 is not coupled to the transmitter 200. FIG. 17 is a longitudinal cross-sectional view illustrating the state of the sensor 240 when the needle assembly 170 is coupled to the transmitter 200.

Referring to FIGS. 16 and 17, the portion between the connection portion and insertion portion of the sensor 240 is placed on the inclined track 253, so that the sensor 240 extending to the outside of the lower housing through the lower through hole 251 forms a first bent portion 241 that is bent at an obtuse angle with respect to of the inclined track 253. When the needle assembly 170 is coupled in this state, one end of the sensor 240, that is, the insertion portion of the sensor 240, is accommodated in the needle 171 and is bent at an obtuse angle, thereby forming a second bent portion 242. That is, the middle portion of the sensor 240 is not directly bent at a right angle, but two bent portions are formed at an obtuse angle between one end and the other end of the sensor 240, and one end and the other end of the sensor 240 form a right angle with the two bent portions formed in this way in between.

Meanwhile, for convenience of explanation, it is illustrated in the present disclosure that the bent portions 241 and 242 are formed by discontinuous surfaces, but it should be noted that they may have a continuously bent shape.

The sensor 240 can be bent because it is made of a flexible film, but when it is bent at a small angle and folded, an electrode of the sensor 240 or a material sputtered on the sensor 240 may be damaged by cracks. When the two bent portions are formed in the sensor 240 in this way, damage to the film constituting the sensor 240 and the material sputtered on the film can be prevented.

In addition, since the sensor 240 forms a predetermined angle downward by the first bent portion 241 before the needle 171 is assembled into the sensor 240, it is easy to assemble the needle 171 coupled to the upper portion of the sensor 240 through the lower portion of the transmitter 200.

Additionally, the tension applied to the electrode can be reduced in a state in which the sensor is inserted into the user's skin and fixed.

The adhesive tape 260 is located on the bottom surface of the lower case 250, and allows the transmitter 200 to be adhered and fixed to the user's skin. An opening is formed in the center of the adhesive tape 260 so that the sensor 240 extending from the lower case 250 can pass through the opening and be placed therein.

Referring to FIG. 3 again, the adhesive tape 260 is configured to be divided according to the position and number of ribs 132a and 132b formed in the cap of the applicator.

In this embodiment, since two ribs 132a and 132b are arranged along the diameter of the cap 130, the cap 130 is configured in an approximately θ shape with a bisected shape when viewed from the bottom or top, so that the adhesive tape 260 is also configured on the bottom surface of the lower case 250 in an approximately θ shape with a bisected center.

Thus, when the adhesive tape 260 divided according to the shape of the ribs 132a and 132b is attached to the bottom surface of the lower case 250, the divided adhesive tapes 260 may be inserted through the right and left openings 131 of the cap 130, respectively.

FIG. 18 illustrates the coupling of a needle assembly and a transmitter according to another embodiment of the present disclosure.
(a) of FIG. 18 is a front perspective view showing the appearance of a needle assembly 170' before it is coupled with a transmitter 200', and (b) of FIG. 18 is a cross-sectional view showing the appearance of a needle assembly 170' coupled with a transmitter 200'.

Referring to (a) of FIG. 18, a needle assembly 170' according to another embodiment includes a needle 171' and a grip portion 172'. Further, a transmitter 200' according to another embodiment may further include a packing portion 235'.

The packing portion 235' is located inside a cylindrical holder 230' located within an upper case 210' and a lower case 250'.

The packing portion 235' is made of an elastic rubber material, and seals the opening at the top of the holder 230'.

Although it is illustrated in FIG. 18 that the packing portion 235' is located on the holder 230' as a separate component, the packing portion 235' may be formed to be integrated with the holder 230' by double injection.

As described above, after the sterilization and assembling process is carried out with the needle 171' of the needle assembly 170' penetrating the packing portion 235' and the applicator is fired by the user, the needle 171' inserted into the holder 230' is separated from the holder 230' while the needle assembly 170' is retracted, and a portion through which the needle 171' penetrated is sealed by the elasticity of rubber.

Thus, when the transmitter 200' is attached and then used, the portion through which the needle 171' penetrated is sealed by the packing portion 235' to be exposed to the outside, and particularly, the inflow of foreign substances such as water is prevented by the hydrophobicity of a rubber material, thereby providing an effect of decreasing a risk of infection.

It will be appreciated by those skilled in the art that the present invention as described above may be implemented into other specific forms without departing from the technical spirit thereof or essential characteristics. Thus, it is to be appreciated that embodiments described above are intended to be illustrative in all aspects, and not restrictive. The scope of the present invention is represented by the appended claims to be described below rather than the detailed description, and it is to be interpreted that the meaning and scope of the appended claims and all the changes or modified forms derived from equivalents thereof come within the scope of the present invention.

## Claims

1. An insertion device assembly comprising:
a transmitter; and
an applicator including the transmitter mounted therein,
wherein the transmitter comprises an upper case; a lower case coupled to the upper case to form an accommodation space therein; a sensor disposed on the lower case; a hollow holder disposed at a center of the lower case and disposed on the sensor; and
wherein the applicator comprises an upper housing having an opening formed at a lower portion thereof and having a button on an outer circumference thereof; a cap configured to be mounted on or separated from the upper housing; a lower housing disposed inside the cap and coupled to an opening at an open lower portion of the upper housing to form an inner space with the upper housing; a needle assembly disposed at an upper portion of the transmitter in the inner space and configured to have at least a portion inserted into the holder; a needle carrier disposed in the inner space and configured to linearly move the needle assembly; and a driving unit configured to provide power to the needle carrier.

2. The insertion device assembly of claim 1,
wherein the needle assembly comprises a needle configured to penetrate a user's skin; a grip portion coupled to one end of the needle carrier; and a needle injection portion disposed between the needle and the grip portion and configured to be frictionally coupled to the holder.

3. The insertion device assembly of claim 2,
wherein the needle injection portion is coupled to the holder when assembling the insertion device assembly, and
the needle injection portion is decoupled from the holder when using the insertion device assembly.

4. The insertion device assembly of claim 3,
wherein the cap comprises an opening formed on a lower surface thereof; a plurality of ribs, at least some thereof being disposed in the lower housing, extending radially inwardly from an inner circumference of the cap; and a needle accommodation portion supported by the plurality of ribs and configured to surround at least a part of the needle.

5. The insertion device assembly of claim 4,
wherein the needle assembly further comprises a cap injection portion having a shape corresponding to a shape of an upper surface of the needle accommodation portion and disposed on the upper surface of the needle accommodation portion, and
the cap injection portion is in contact with a lower surface of the lower case.

6. The insertion device assembly of claim 4,
wherein the plurality of ribs are two or more and are disposed symmetrically with respect to a center of the needle accommodation portion,
the transmitter further comprises an adhesive tape attached to the lower surface of the lower case, the number of which is equal to the number of the plurality of ribs, and
the adhesive tape is inserted through the opening.

7. The insertion device assembly of claim 1,
wherein the transmitter further comprises a PCB (Printed Circuit Board) accommodated in the accommodation space between the upper case and the lower case and a dummy PCB connecting the sensor and the PCB.

8. The insertion device assembly of claim 1,
wherein the transmitter further comprises a packing portion located on the holder and made of an elastic material.

9. The insertion device assembly of claim 2,
wherein the transmitter comprises a lower opening located in the lower case and provided with the holder located therein; and an inclined track having an inclined surface formed to be inclined downward from one side of the lower opening, a part of the sensor being placed on the inclined surface.

10. The insertion device assembly of claim 9,
wherein the insertion portion of the sensor is located in the needle penetrating the holder, and
two bent portions forming an obtuse angle are formed between one end of the sensor and the other end thereof by the inclined surface and the needle.

11. An insertion device assembly comprising:
a transmitter; and
an applicator configured to be mounted therein with the transmitter,
wherein the insertion device assembly is divided into a first assembly to a fourth assembly according to an assembling order, and
the first assembly comprises a cap comprising an open upper portion and an open lower portion; a lower housing disposed in the cap; a lower case of the transmitter, the lower case being disposed on at least a part of the lower housing; a sensor disposed on the lower case; a hollow holder disposed at a center of the lower case and disposed on the sensor; and a needle assembly disposed on the transmitter and configured to allow at least a part thereof to be inserted into the holder.

12. The insertion device assembly of claim 11,
wherein the second assembly further comprises, in the first assembly sterilized with E-beam, a PCB disposed on the lower case; and an upper case coupled to the lower case to form an accommodation space therein.

13. The insertion device assembly of claim 12,
wherein the third assembly further comprises, in the second assembly, a needle carrier configured to linearly move the needle assembly; and a driving unit configured to provide power to the needle carrier.

14. The insertion device assembly of claim 13,
wherein the fourth assembly further comprises, in the third assembly, an adhesive tape attached to a lower surface of the lower case; and a sealing portion configured to seal the open lower portion of the cap.

15. The insertion device assembly of claim 13,
wherein the needle assembly comprises a needle configured to penetrate a user's skin; a grip portion coupled to one end of the needle carrier; and a needle injection portion disposed between the needle and the grip portion and configured to be frictionally coupled to the holder.

16. The insertion device assembly of claim 15,
wherein the cap comprises a plurality of ribs, at least some thereof being disposed in the lower housing, extending radially inwardly from an inner circumference of the cap; a needle accommodation portion supported by the plurality of ribs and configured to surround at least a part of the needle; and a cap injection portion having a shape corresponding to a shape of an upper surface of the needle accommodation portion and disposed on the upper surface of the needle accommodation portion, and
the cap injection portion is in contact with a lower surface of the lower case.

17. The insertion device assembly of claim 16,
wherein the plurality of ribs are two or more and are disposed symmetrically with respect to a center of the needle accommodation portion,
the transmitter further comprises an adhesive tape attached to the lower surface of the lower case, the number of which is equal to the number of the plurality of ribs, and
the adhesive tape is inserted through the open lower portion of the cap.

18. An insertion device assembly comprising:
a transmitter; and
an applicator including the transmitter mounted therein,
wherein the applicator comprises an upper housing having an opening formed at a lower portion thereof and having a button on an outer circumference thereof; a cap configured to be mounted on or separated from the upper housing, with the upper portion and lower portion of the cap being open; a lower housing disposed inside the cap and coupled to an opening at an open lower portion of the upper housing to form an inner space with the upper housing; and a needle assembly comprising a needle configured to penetrate the transmitter,
wherein the cap comprises a plurality of ribs, at least some thereof being disposed in the lower housing, extending radially inwardly from an inner circumference of the cap; a needle accommodation portion supported by the plurality of ribs and configured to surround at least a part of the needle; and a cap injection portion having a shape corresponding to a shape of an upper surface of the needle accommodation portion, disposed on the upper surface of the needle accommodation portion and being in contact with a lower surface of the transmitter.

19. The insertion device assembly of claim 18,
wherein the plurality of ribs are two or more and are disposed symmetrically with respect to a center of the needle accommodation portion,
the transmitter further comprises an adhesive tape attached to the lower surface of the transmitter, the number of which is equal to the number of the plurality of ribs, and
the adhesive tape is inserted through the open lower portion of the cap.
